# EUROPEAN PATENT APPLICATION

(11) **EP 3 943 932 A1**
(43) Date of publication of application: **26.01.2022**
(21) Application number: 20779207.8
(22) Date of filing: 19.03.2020
(51) Int. Cl.: G01N 33/15, A61K 35/17, A61P 35/00, A61P 37/04, G01N 33/48

(54) **METHOD FOR PROVIDING IMMUNE CELLS**

(30) Priority: 22.03.2019 JP 2019054249
(71) Applicant: GAIA BioMedicine Inc., Chuo-ku Fukuoka-shi Fukuoka 810-0023 (JP); Yonemitsu, Yoshikazu, Fukuoka-shi, Fukuoka 810-0023 (JP)
(72) Inventor: HARADA, Yui, Fukuoka-shi, Fukuoka 819-0395 (JP); KURAMORI, Kazuyuki, Fukuoka-shi, Fukuoka 810-0023 (JP); YONEMITSU, Yoshikazu, Fukuoka-shi, Fukuoka 810-0023 (JP)
(74) Representative: Vossius & Partner Patentanwälte Rechtsanwälte mbB
(86) International application number: PCT/JP2020/012386
(87) International publication number: WO 2020/196297

(57) **Abstract**

An object is to quickly provide immune cells to a recipient. A system for providing immune cells obtained from a donor (non-recipient) to a recipient, which comprises the following steps: the step of preparing a formulation of immune cells collected from a donor in a unit suitable for administration; the step of performing an evaluation test on the formulation; the step of refrigerating or freezing the formulation and stocking it with immune type information of the donor; and the step of selecting a formulation suitable for the recipient from stocked formulations subjected to the evaluation test or identifying a recipient suitable for one selected stocked formulation subjected to the evaluation test.

## Description

### Technical Field

The present invention relates to a method for quickly providing immune cells to a recipient.

### Background Art

Cancer cells produced in the body are eliminated by the immune system, but cancer cells that cannot be eliminated for some reason proliferate and eventually develop cancer. As the cancer therapy, surgery, anticancer drugs (chemotherapy), and radiation therapy are the most common, but recently immunotherapy is attracting attention. Immunotherapy is a method of treating cancer by utilizing the power of immunity, and is a concept that includes various treatment methods. Cell therapy (cellular immunotherapy) is one type of immunotherapy, in which immune cells are activated or proliferated outside the body, and transplanted into patients. The cells that play a central role in the immunity are monocytes, neutrophils, eosinophils, basophils, T cells, B cells, natural killer (NK) cells, natural killer T (NKT) cells, and dendritic cells contained in the blood, which are collectively called immune cells.

Concerning these, the most advanced cell therapy is that using T cells, which are relatively easily proliferated outside the body. Tumor Infiltrating Lymphocyte (TIL) therapy, which one of the T-cell-based cell therapies, involves in vitro large scale cultivation of lymphocytes isolated from surgically resected cancerous tissues of patients or thoraco-abdominal fluid collected from patients, and their transplantation into patients. It has been reported to be effective for melanoma in many patients, and applications thereof to cervical cancer, lung cancer, and so forth are expected.

### Summary of the Invention

### Object to be Achieved by the Invention

T cells have the ability to recognize and attack specific antigens. Therefore, T cell-based therapies use T cells that can specifically react with the cancer of a patient to be treated. In other words, T-cell therapy is an individualized treatment and is not versatile.

In addition, when transplanting cells into a patient, the first consideration is to use cells collected from the patient himself or herself to avoid rejection. However, depending on the patient's condition, it may be difficult to collect the necessary amount of cells for the treatment. Further, the degree to which cells can be activated and proliferated outside the body varies from person to person, and there are cases in which such activation and proliferation are difficult. In addition, since it takes a certain period of time to activate and proliferate cells, treatment cannot be started immediately. In this regard, there has also been proposed use of immune cells differentiated and proliferated from iPS cells of other people, which are less prone to be rejected. However, with regard to iPS cells, it is necessary to manage introduction of foreign genes during the creation process thereof and reduction of the risk of cancerization after induction of differentiation.

### Means for Achieving the Object

The inventors of the present invention propose the following inventions as a solution to the aforementioned problems.
[1] A method for providing immune cells obtained from a donor (non-recipient) to a recipient, which comprises the following steps:
   the step of preparing a formulation of immune cells collected from a donor in a unit suitable for administration;
   the step of performing an evaluation test on the formulation;
   the step of refrigerating or freezing the formulation and stocking it with immune type information of the donor; and
   the step of selecting a formulation suitable for the recipient from stocked formulations subjected to the evaluation test or identifying a recipient suitable for one selected stocked formulation subjected to the evaluation test on the basis of immune type information of the donor and immune type information of the recipient.
      The system can be rephrased as method or apparatus.
[2] The system according to 1, which includes the step of proliferating and/or activating the immune cells collected from the donor prior to the step of preparing the formulation.
[3] The method according to 1 or 2, wherein the step of preparing the formulation is carried out by storing one unit of the immune cells in a resin bag.
[4] The system according to any one of 1 to 3, wherein the one unit consists of 1.0 × 10⁷ to 1.0 × 10¹⁰ cells.
[5] The system according to any one of 1 to 4, wherein the evaluation test includes a sterility test.
[6] The system according to any one of 1 to 5, wherein the evaluation test includes a test for evaluating performance of the cells.
[7] The system according to any one of 1 to 6, wherein the step of making the formulation into a form suitable for intravenous drip infusion is carried out by diluting the formulation with an isotonic solution.
[8] The system according to any one of 1 to 7, wherein the immune cells are NK cells, and the recipient is a cancer patient.
[9] The system according to any one of 1 to 8, wherein the donor is a healthy person.
[10] The system according to 2, wherein the step of proliferation and/or activation comprises mixing immune cells collected from multiple donors and proliferating and/or activating them.

### Brief Description of the Drawings

[Fig. 1] Types of immune cells contained in the blood.
[Fig. 2] A schematic diagram of an exemplary method for extracting immune cells from the blood.
[Fig. 3] A schematic diagram of an example of the system of the present invention. D represents a donor, and R represents a recipient.

### Modes for Carrying out the Invention

The present invention provides a system for providing immune cells obtained from a donor (non-recipient) to a recipient, which comprises the following steps:
the step of preparing a formulation of immune cells collected from a donor in a unit suitable for administration;
the step of performing an evaluation test on the formulation;
the step of refrigerating or freezing the formulation and stocking it with immune type information of the donor;
the step of selecting a formulation suitable for the recipient from stocked formulations subjected to the evaluation test or identifying a recipient suitable for one selected stocked formulation subjected to the evaluation test on the basis of immune type information of the donor and immune type information of the recipient; and
the step of making the selected formulation into a form suitable for intravenous drip infusion.

### [Preparation of formulation]

In the present invention, immune cells extracted from each donor are formulated in a unit suitable for administration.

The present invention is characterized in that the immune cells are collected beforehand from a donor and not from the patient himself or herself, who is the subject of the immunotherapy. In other words, according to the present invention, the donor is not a recipient (non-recipient). The donor is preferably a healthy person, and the recipient may be a cancer patient.

The invention is also characterized by the use of cells "collected" from a donor. The term "collected" cells refers to cells obtained without any genetic modification, and excludes cells that have undergone genetic manipulation, such as iPS cells.

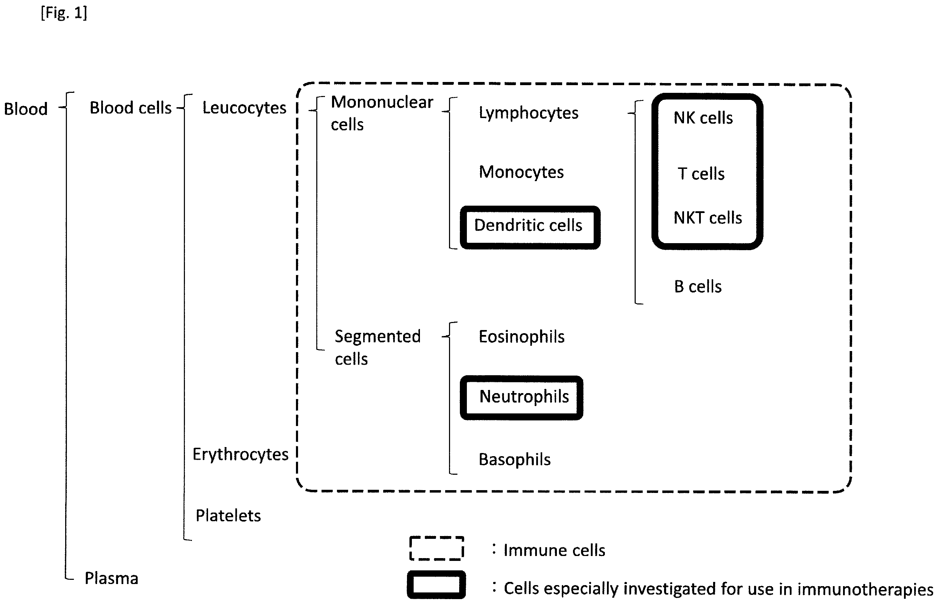

Neutrophils, NK cells, T cells, NKT cells, and dendritic cells can be mentioned as cells that have been conventionally considered for use in immune cell therapy (see Fig. 1). According to a preferred embodiment of the present invention, NK cells are used as the immune cells. NK cells are able to distinguish between normal and abnormal cells, and can attack abnormal cells such as cancer cells and virus-infected cells. In this respect, NK cells can attack a wider range of cells compared with T cells, which recognize specific cells in an antigen-specific manner.

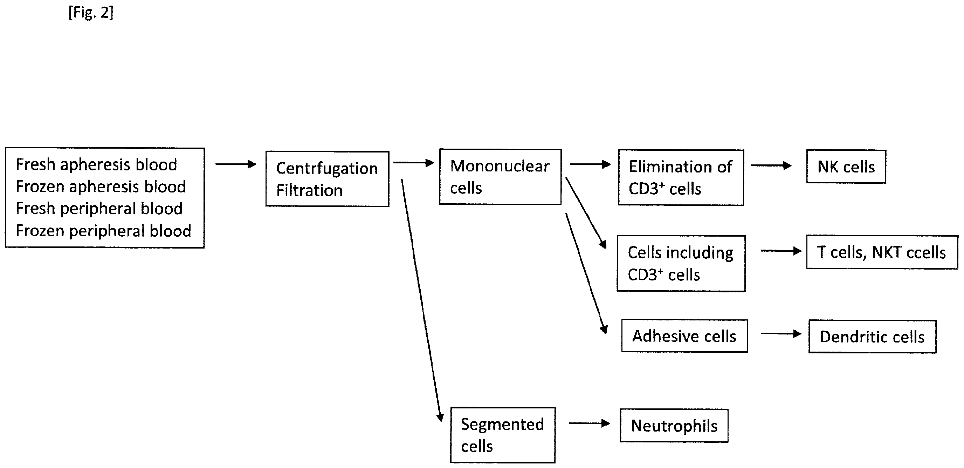

Immune cells can be obtained by obtaining mononuclear cells by centrifugation or filtration separation from blood cells, and so forth collected from peripheral blood, cord blood, bone marrow and/or lymph nodes, and removing unwanted cells from the obtained mononuclear cells. According to a particularly preferred embodiment of the present invention, the raw material for obtaining mononuclear cells is peripheral blood, and the peripheral blood may be collected by the apheresis method. The peripheral blood may be fresh or cryopreserved (see Fig. 2).

Immune cells are suspended in a liquid suitable for refrigeration or cryopreservation, and made into a formulation. The number of immune cells contained in a single formulation should be appropriate for administration. For example, the number of cells to be administered in a single session of cell therapy can be defined as one unit, and 1/3, 1/2, or 1-fold amount of the unit can be used for one formulation. One unit consists of, for example, 1.0 × 10⁷ or more of cells, or may consist of 5.0 × 10⁷ or more of cells, or 1.0 × 10⁸ or more of cells. The maximum amount can be 1.0 × 10¹⁰ or less of cells in any case, and may be 5.0 × 10⁹ or less of cells, or 1.0 × 10⁹ or less of cells.

The solution used for suspending the immune cells (suspension medium) may be an isotonic or hypertonic solution. If the formulation is to be cryopreserved, it is preferable to use a hypertonic solution containing inorganic salts and a cytoprotective agent such as dimethyl sulfoxide (DMSO), ethylene glycol, povidone-iodine, or trehalose. The isotonic solution is a solution of which osmolarity is approximately equal to the osmolarity of plasma (285 ± 5 mOsm/L). The hypertonic solution is a solution of which osmolarity is higher than that of plasma. When cells are suspended in a hypertonic solution containing a cytoprotective agent, it is preferable to start the freezing process within 15 minutes after suspension. When a hypertonic solution is used, the formulation is diluted to lower the osmotic pressure thereof at the time of intravenously administering the formulation (see below).

The formulation should be preferably performed under conditions that conform to the regulations for manufacturing and quality control of pharmaceuticals and quasi-pharmaceuticals (Good Manufacturing Practice, GMP) and the standards for manufacturing and quality control of products for regenerative medicine etc. (Good Gene, Cellular, and Tissue-based Products Manufacturing Practice, GCTP).

### [Proliferation and/or activation]

According to a preferred embodiment of the present invention, a step of proliferating and/or activating the immune cells is carried out prior to the formulation step. According to a particularly preferred embodiment, the immune cells are proliferated and activated. The term "proliferating and/or activating" means to carry out at least one of proliferation and activation.

Proliferation and/or activation of immune cells is performed by, for example, incubation at 37°C and 5% CO₂ in a saturated water vapor atmosphere using a suitable medium. A longer proliferation period provides more immune cells, and is therefore advantageous. The proliferation period is not particularly limited, but can be, for example, 7 to 28 days, and it may be 10 to 18 days or 12 to 16 days, for example, 14 days.

Effective conditions for the proliferation and activation of NK cells include use of a medium suitable for proliferation of NK cells. Examples of such a medium include, but not limited to, KBM501 medium (Kohjin Bio Co., Ltd.), CellGro SCGM medium (CellGenix, Iwai Chemical Co., Ltd.), X-VIVO15 medium (Lonza, Takara Bio Inc.), IMDM, MEM, DMEM, RPMI-1640, and so forth. Interleukin 2 (IL-2) may be added to the medium at such a concentration that the purpose of the present invention can be achieved. The concentration of IL-2 may be higher than 2000 IU/mL, and may be in the range of 2500 to 2813 IU/mL. IL-2 preferably has the human amino acid sequence thereof, and is preferably produced by a recombinant DNA technique for safety reasons. The concentration of IL-2 may be expressed in Japan reference unit (JRU) and international unit (IU). Since 1 IU is about 0.622 JRU, 1,750 JRU/mL is about 2,813 IU/mL. Human AB serum, available from BioWhittaker and others, or donated human serum albumin, available from the Japanese Red Cross Society, may be added to the medium. Human AB serum is preferably added at a concentration of 1 to 10%, and donated human serum albumin is preferably added at a concentration of 1 to 10%. In addition, a composition formulated for the proliferation of immune cells may be added to the medium instead of or together with serum. Such a composition is commercially available. For example, the UltraGro series (AventaCell) and CTS Immune Cell SR (Thermo Fisher Scientific) can also be used for the present invention. The medium may contain appropriate proteins, cytokines, antibodies, chemical compounds, and other components, provided that they do not impair the NK cell proliferating effect. Cytokines may include interleukin 3 (IL-3), interleukin 7 (IL-7), interleukin 12 (IL-12), interleukin 15 (IL-15), interleukin 18 (IL-18), interleukin 21 (IL-21), stem cell factor (SCF), and/or FMS-like tyrosine kinase 3 ligand (Flt3L). IL-3, IL-7, IL-12, IL-15, IL-18, IL-21, SCF, and Flt3L preferably have human amino acid sequences thereof, and are preferably produced by a recombinant DNA technique for safety reasons.

According to a preferred embodiment of the present invention, the immune cells are subjected to an activation operation using some cytokine in vitro. The immune cells that are subjected to such activation become NK cells having high cytotoxic activity (highly active NK cells), etc. The activation operation is typically based on incubation of the cells in a medium containing interleukin (IL) 2.

Such highly active NK cells, etc. include those defined in [1], [2], [3], and [4] mentioned below.

[1] NK cells having the following characteristics (1) and (2):
(1) CD16-positive, high CD56 expression, and CD57-negative.
(2) NKG2C-positive, NKG2A-negative or low NKG2A expression, and CD94-positive.

The highly active NK cells of [1] may have high CD16 expression. The highly active NK cells of [1] may further have the following characteristics irrespective of whether they have high CD 16 expression. (3) Cytotoxic activity of 50% or higher as observed in co-culture of the NK cells as effector cells (E), and K562 cells as target cells (T) at a mixing ratio (E:T) of 1:1.

The highly active NK cells of [1] can also be expressed as follows:
NK cells that can be obtained by 14-day culture of cell population prepared by eliminating CD3-positive cells from mononuclear cells derived from peripheral blood of a healthy person using CD3 beads (e.g., CliniMACS CD3, Miltenyi Biotech, catalog number 130-017-601), LD column (e.g., Miltenyi Biotech, Catalog No. 130-042-901) and separation buffer (e.g., PBS containing 0.5% human AB serum (inactivated) and 2 mM EDTA) in an appropriate medium (e.g., Cosmedium 008 supplemented with 5% human AB serum (inactivated)), and have the following characteristics (1) and (3):
(1) CD16-positive, high CD56 expression, and CD57-negative.
(3) Cytotoxic activity of 50% or higher as observed in co-culture of the NK cells as effector cells (E), and K562 cells as target cells (T) at a mixing ratio (E:T) of 1:1.

For details of the characteristics and more specific production methods of the highly active NK cells of [1], Japanese Patent Unexamined Publication (Kokai) No. 2018-193303 can be referred to.

[2] The following cells:
cells that are CCR5-positive, CCR6-positive, CXCR3-positive and CD3-negative.

The cells of [2] may also have high CD11c expression.

The cells of [2] may also be represented as follows:
cells that are CCR5-positive, CCR6-positive, CXCR3-positive, integrin α1-positive, integrin α3-positive, integrin β3-negative, and CD3-negative, or cells that are CCR5-positive, CCR6-positive, and CXCR3-positive, have high CD11a expression, and high CD11c expression, and are CD3-negative, wherein the high expressions are determined by comparison with expression observed in a substantially uncultured population of NK cells obtained from peripheral blood.

According to the examinations of the inventors of the present invention, the cells of [2] exhibit extremely high cytotoxic activity against solid cancers that have formed a tumor mass. For details of the characteristics of the cells of [2] and more specific production methods thereof, Japanese Patent Unexamined Publication (Kokai) No. 2019-170176 can be referred to.

[3] Highly active NK cells that are obtainable by the following method.

To mononuclear cells obtained from fresh peripheral blood or frozen apheresis blood, CD3 beads (e.g., CliniMACS CD3, Miltenyi Biotech, 130-017-601 (5 µL per 1 × 10⁷ cells)) are added, and CD34 beads (e.g., CliniMACS CD34, Miltenyi Biotech, 130-017-501 (2.5 µL per 1 × 10⁷ cells)) are further added if frozen apheresis blood is used, and the cells are suspended, and incubated at 4°C for 15 minutes, then a separation buffer (e.g., PBS containing 0.5% human AB serum (inactivated at 56°C for 30 minutes), and 2 mM EDTA) is added to the cells to sufficiently suspend them, and the suspension is centrifuged. The supernatant is removed, and the cells are suspended in 0.5 mL of the separation buffer to achieve a cell count of up to 1 × 10⁸ cells per LD column (e.g., Miltenyi Biotech, 130-042-901). After 2 mL of the separation buffer is added beforehand, the cell suspension is added to the LD column, and the eluate from the LD column is collected. The separation buffer (1 mL) is further added to the LD column, and the eluate is collected. The collected eluates are centrifuged, the supernatant is removed, and then the cells are suspended in an appropriate medium (e.g., KBM501 medium containing either 5% human AB serum (inactivated at 56°C for 30 minutes) or 5% UltraGRO (AventaCell, HPCPLCRL10) containing 2 U/mL sodium heparin) at a density of 5 × 10⁵ cells/mL when peripheral blood is used, or 1 × 10⁶ cells/mL when frozen apheresis blood is used, and cultured up to day 14 with changing the medium as necessary.

For the specific production method of the highly active NK cells of [3], Japanese Patent Application No. 2020-035297 (unpublished at the time of filing of this application), the section of Examples can be referred to.

[4] Cells that can be obtained by culture for obtaining the cells of [1] to [3] with addition of any one selected from the group consisting of IL-12, IL-15, and IL-18 in addition to or instead of IL-2 at such a concentration that the purpose of the invention can be achieved. For specific methods for producing such cells, Leong JW et al., Biol. Blood Marrow Transplant., 20 (2014) 463-473 can be referred to.

When proliferating and/or activating NK cells, cell materials collected from multiple donors may be mixed. According to the investigations of the inventors of the present invention, it has been revealed that proliferation of NK cells is improved by mixing materials collected from multiple donors.

Containers for proliferating and/or activating immune cells include, but are not limited to, commercially available dishes, flasks, plates, multi-well plates, and bags. It is preferable to use a container that allows proliferation of a large number of cells and easy handling. An example of such a container is a cell proliferation bag made of a material with high gas permeability.

If a bag is used, it is preferable to invert it upside down during the proliferation and/or activation period. A part of the immune cells may adhere to the bottom of the container. In general, proliferation efficiency of adhesive cells is greatly influenced by the number of cells per volume as well as the number of cells per unit area to which the cells in the container can adhere. By inverting the bag during the proliferation and/or activation period of the immune cells, the surface that was previously on the upper side and therefore had relatively little cell adhesion can also be effectively utilized, which allows more efficient proliferation.

### [Stocking]

Formulated immune cells are refrigerated (e.g., 4°C) or frozen (e.g., about - 196°C) and stocked with donor immune type information. As equipment for refrigeration or freezing, conventional equipments used for similar purposes such as equipments for refrigeration and freezing of blood preparations can be utilized.

Immune type information refers to information relating to immunity, such as that concerning ABO blood type, RH blood type, HLA type, and KIR type. Immune type information is used for the selection of stock formulations described below. A formulation containing immune cells obtained from a certain donor should be stocked in such a manner that the immune type information obtained from the same donor is associated with the formulation. They can be associated by, for example, assigning a unique number to each donor, and assigning that number to both the formulation and the immune type information. The immune type information and the number can be documented or digitized. The information to be attached to the formulation to be stocked may also include the results of the evaluation test described below.

### [Evaluation test]

According to the present invention, the formulation is subjected to an evaluation test for a cell formulation. The evaluation test can be performed in conjunction with a treatment for stocking the formulation.

The evaluation test includes safety test and performance test (test for evaluating performance of the cells). The safety test includes sterility test, endotoxin test, mycoplasma-negative test, and virus-negative test. Among such evaluation tests, those essential for quality assurance as a drug product should preferably be completed prior to the step of selecting a formulation suitable for a recipient or identifying a recipient suitable for one selected stock formulation subjected to the evaluation tests, which step will be described below. In particular, it is preferable to complete the sterility test, which requires a long testing period, because rapid provision of immune cells to the recipient can be thereby ensured.

The performance test includes cytotoxic activity test. Cytotoxic activity refers to the ability of the subject immune cells (effector cells, E) to damage the target cells (T), unless otherwise stated. Cytotoxic activity can be expressed as percentage (%) of the target cells killed by the effector cells and is calculated in accordance with the following equation.

### (Cell death observed in co-culture with effector cells - Natural cell death (negative control))/(Maximum cell death (positive control) - Natural cell death (negative control)) × 100

When measuring cytotoxic activity, in general, the mixing ratio of effector cells and target cells (E:T) and the time of co-culture of effector cells and target cells may be appropriately determined depending on the type of cells used and strength of the activity thereof, as well as the degree of cytotoxic activity of the effector cells, etc. When NK cells are used as the effector cells, the target cells may be, but are not limited to, the K562 cells, acute myeloid leukemia cells, or chronic myeloid leukemia cells.

The effector cells and target cells, and living cells and dead cells can be distinguished and quantified by using reagents such as antibodies labeled with a radioactive substance, fluorescent dye, or the like. The cytotoxic activity of NK cells as effector cells can be measured by, for example, using the K562 cells as target cells at E:T ratio of 1:0.05 to 10, preferably 1:0.1 to 5, and incubating them for 0.5 to 18 hours, preferably 1 to 12 hours.

In the case of NK cells, when the K562 cells are used as the target cells, and the cells are mixed at E:T ratio of 1:1, and co-cultured for 1 to 3 hours, more specifically, 2 hours, the cytotoxic activity should be 50% or higher, preferably 60% or higher, more preferably 70% or higher.

### [Selection of stock formulation, etc.]

According to the present invention, a suitable formulation for a recipient is selected from stocked formulations subjected to the evaluation test on the basis of the immune type information of the donor and the immune type information of the recipient. Alternatively, a recipient suitable for one selected stock formulation subjected to the evaluation test can be identified.

The selection based on immune type information can be performed in consideration of various conditions. For example, the HLA type information of the donors attached to the stock formulations can be compared with the HLA type information of the recipient, and a stock formulation with a high matching degree (evaluated in advance) can be selected, or a recipient with a high matching degree for a stock formulation can be identified.

In the case of NK cells, selection based on KIR ligand mismatch may lead to better outcomes with respect to relapse, graft-versus-host disease (GvHD), and prognosis due to the allogeneic antigen reactivity of NK cells. For example, it is known that bone marrow transplantation for hematological cancers provides better prognosis when the KIR ligand mismatch is high (Symons HJ, et al., Biol. Blood Marrow Transplant., 2010). The same phenomenon has been reported for a case that the subject is a solid tumor (Leung W, et al., Br. J. Cancer, 2007). Stocks of immune types expected to be highly effective for HLA-C1/C1, which accounts for 86% of the Japanese population, or stocks of a wide variety of immune types will increase not only the speed but also the versatility of the treatment.

### [Preparation of injection for intravenous drip infusion]

According to the present invention, the selected formulation can be made into a form suitable for intravenous injection into the recipient. This is usually attained by diluting the formulation with a liquid suitable for intravenous administration, such as an isotonic solution. Examples of such a liquid include, for example, saline, phosphate-buffered saline (PBS), lactated Ringer's solution, Plasma-Lyte A, and so forth. Such a pharmaceutical composition or solution may also contain a pharmaceutically acceptable additive.

When an injection is prepared, antibodies may be added if the immune cells are NK cells. Many of antibody drugs are said to exhibit antitumor effects through ADCC (antibody-dependent cellular cytotoxicity) activity after they are intravenously administered. However, in addition to NK cells, monocytes/macrophages and neutrophils are also recruited when the ADCC activity is exhibited. And the effectors other than NK cells exhibit the ADCC activity without distinguishing between normal cells and cancer cells, which is thought to be also responsible for the occurrence of side effects. In the present invention, NK cells and antibodies may be mixed before administration, so that the NK cells have the antibodies in advance. The effectors are thereby limited to the NK cells, therefore the amount of antibody to be administered can be reduced, and this is considered to be extremely effective also in reducing side effects.

### [Treatment]

According to the present invention, the prepared intravenous infusion is dripped into the vein of the recipient to treat the recipient's disease. If the immune cells are NK cells and the recipient is a cancer patient, the administration of the prepared intravenous drip infusion will cause the NK cells to damage the cancer cells in the recipient's body.

### [Advantages of the system of the present invention]

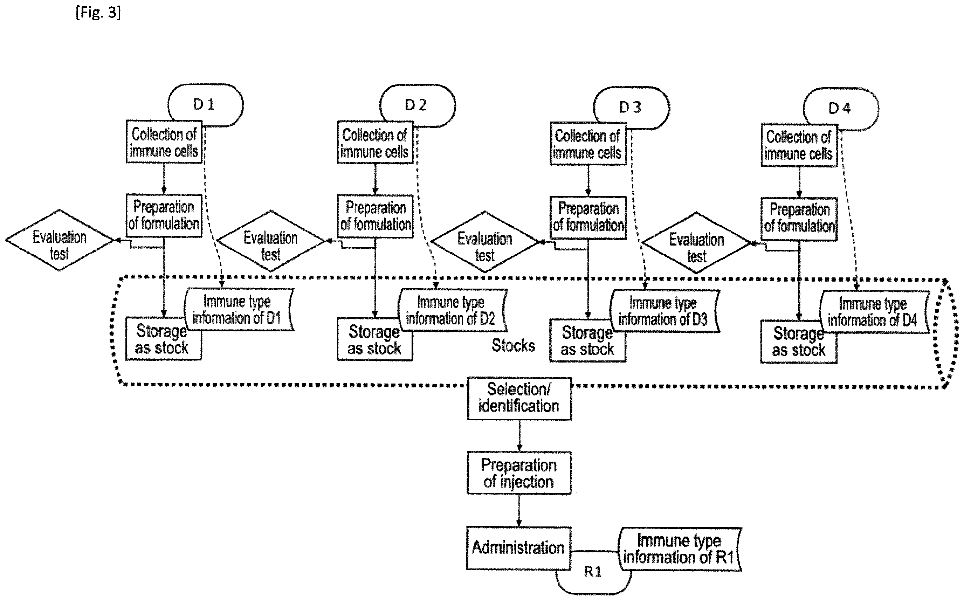

A schematic diagram of an example of the system of the present invention is shown as Fig. 3. According to the system of the present invention, immune cells are stocked, and thereby the immune cells can be rapidly provided to the recipient. The system of the present invention also has versatility. The system of the present invention is particularly suitable for providing formulations containing NK cells.

Conventional T-cell therapies are individualized treatments and lack versatility. Since T cells have the ability to recognize and attack specific antigens on autologous HLA (human leukocyte antigen), conventional T cell-based therapies use T cells that can specifically react with the cancer of the patient to be treated. In addition, when there are used CAR-T cells having an ability to recognize and attack specific antigens on the cell membrane rather than antigens on the autologous HLA, in principle, autologous T cells are used to deter GvHD. As a solution for this problem, techniques of using operation for modifying or removing such genes as HLA and TCR (T cell receptor, T cells recognize antigens through TCR) genes in addition to the introduction of CAR are being developed, but none of these have yet been put into practical use.

### Industrial Applicability

The system provided by the present invention can rapidly provide immune cells to a recipient, and therefore it has potential for use in medicine and other fields. The ability to rapidly provide immune cells is considered to be extremely useful for patients with malignant tumors that have a poor life prognosis and require comparatively high urgency.

## Claims

1. A method for providing immune cells obtained from a donor (non-recipient) to a recipient, which comprises the following steps:
preparing a formulation of immune cells collected from a donor in a unit suitable for administration;
performing an evaluation test on the formulation;
refrigerating or freezing the formulation and stocking it with immune type information of the donor; and
selecting a formulation suitable for the recipient from stocked formulations subjected to the evaluation test or identifying a recipient suitable for one selected stocked formulation subjected to the evaluation test on the basis of immune type information of the donor and immune type information of the recipient.

2. The method according to claim 1, which includes the step of proliferating and/or activating the immune cells collected from the donor prior to the step of preparing the formulation.

3. The method according to claim 1 or 2, wherein the step of preparing the formulation is carried out by storing one unit of the immune cells in a resin bag.

4. The method according to any one of claims 1 to 3, wherein the one unit consists of 1.0 × 10⁷ to 1.0 × 10¹⁰ cells.

5. The method according to any one of claims 1 to 4, wherein the evaluation test includes a sterility test.

6. The method according to any one of claims 1 to 5, wherein the evaluation test includes a test for evaluating performance of the cells.

7. The method according to any one of claims 1 to 6, wherein the step of making the formulation into a form suitable for intravenous drip infusion is carried out by diluting the formulation with an isotonic solution.

8. The method according to any one of claims 1 to 7, wherein the immune cells are NK cells, and the recipient is a cancer patient.

9. The method according to any one of claims 1 to 8, wherein the donor is a healthy person.

10. The method according to claim 2, wherein the step of proliferation and/or activation comprises mixing immune cells collected from multiple donors and proliferating and/or activating them.
